(19) Europäisches Patentamt
European Patent Office
Office européen des brevets

(11) Veröffentlichungsnummer: **0 013 258**
**B1**

(12) **EUROPÄISCHE PATENTSCHRIFT**

(45) Veröffentlichungstag der Patentschrift: **07.07.82**

(51) Int. Cl.³: **C 07 D 303/20** // C08G59/24

(21) Anmeldenummer: **79810187.9**

(22) Anmeldetag: **21.12.79**

(54) **Propenylsubstituierte Phenolglycidyläther, Verfahren zu ihrer Herstellung und ihre Verwendung.**

(30) Priorität: **29.12.78 CH 13280/78**

(43) Veröffentlichungstag der Anmeldung: **09.07.80 Patentblatt 80/14**

(45) Bekanntmachung des Hinweises auf die Patenterteilung: **07.07.82 Patentblatt 82/27**

(84) Benannte Vertragsstaaten: **CH DE FR GB**

(56) Entgegenhaltungen:
**FR-A 1 127 906**
**FR-A 1 306 793**
**US-A 3 466 376**

(73) Patentinhaber: **CIBA-GEIGY AG, Patentabteilung Postfach, CH-4002 Basel (CH)**

(72) Erfinder: **Zahir, Sheik Abdul-Cader, Dr., Elsternstrasse 12, CH-4104 Oberwil (CH)**
Erfinder: **Eldin, Sameer H., Dr., Am Stausee 27/18, CH-4127 Birsfelden (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Propenylsubstituierte Phenolglycidyläther, Verfahren zu ihrer Herstellung und ihre Verwendung

Gegenstand der vorliegenden Erfindung sind propenylsubstituierte Glycidyläther von zweikernigen Phenolen sowie die Isomerengemische aus propenylsubstituierten und allylsubstituierten Phenolglycidyläthern, Verfahren zu ihrer Herstellung und die Verwendung dieser Verbindungen oder Isomerengemische zur Herstellung von Polymeren.

Phenolglycidyläther, welche neben einer oder mehreren Epoxidgruppen noch eine oder mehrere Alkenylgruppen im Molekül enthalten, sind bekannt.

In der USA-A Nr. 3466376 wird der allyl- oder propenylsubstituierte Phenylglycidyläther als Ausgangssubstanz zur Herstellung von entsprechend alkenylsubstituiertem Phenoxy-2-hydroxy-3-isopropylaminopropan verwendet, das für pharmazeutische Formulierungen geeignet ist.

In der FR-A Nr. 1306793 wird unter anderem das Verfahren zur Herstellung des p-Propenylphenylglycidyläthers beschrieben und dessen Verwendung für organische Synthesen, zum Beispiel zur Herstellung von Weichmachern, Stabilisatoren und Schädlingsbekämpfungsmitteln, vorgeschlagen.

In der FR-A Nr. 1127906 wird unter anderem die Herstellung von propenyl- oder alkylsubstituierten Phenylglycidyläthern und deren Verwendung zur Herstellung von Homo- und Copolymeren beschrieben. Während die Homopolymerisate neben relativ geringen Anteilen an niedermolekularen Oligomeren noch grosse Anteile an Monomeren aufweisen, bestehen die Copolymerisate aus höhermolekularen Verbindungen. Die aus der FR-A Nr. 1127906 bekannten alkenylsubstituierten Phenylglycidyläther stellen somit im wesentlichen reaktive Verdünner dar.

In der DE-A Nr. 2726821 werden zum Beispiel Epoxidharzmischungen auf Basis von allylsubstituierten Phenolglycidyläthern beschrieben, die sowohl Maleinimide als auch Härtungsmittel für Epoxidharze enthalten. In „Journal of Applied Polymer Science", Bd. IV, Nr. 11, S. 144, Tabelle 2, werden unter anderem auch die mechanischen Eigenschaften von mit m-Phenylendiamin gehärteten allylsubstituierten Phenolglycidyläthern angegeben.

Es wurde nun gefunden, dass man Polymere mit vergleichsweise besseren mechanischen Eigenschaften erhält, wenn man anstelle der allylsubstituierten Phenolglycidyläther die isomeren propenylsubstituierten Phenolglycidyläther oder die Isomerengemische aus propenyl- und allylsubstituierten Phenolglycidyläthern verwendet.

Gegenstand der vorliegenden Erfindung sind somit Phenolglycidyläther der Formel I:

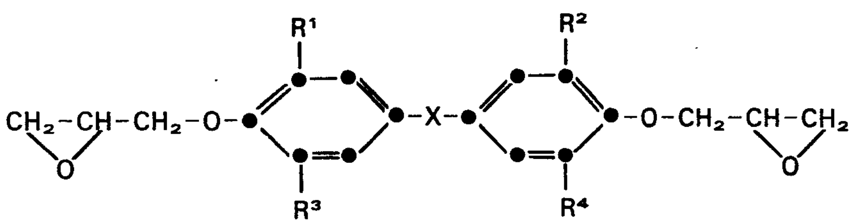

(I)

worin $R^1$, $R^2$, $R^3$ und $R^4$ je ein Wasserstoffatom, eine Allyl- oder Propenylgruppe bedeuten, wobei mindestens einer der Substituenten $R^1$ bis $R^4$ für die Propenylgruppe steht, und X Isopropylen, Methylen, Sulfonyl, $-O-$ oder $-S-$ bedeutet.

Von den Phenolglycidyläthern der Formel I sind besonders solche interessant, die der Formel II:

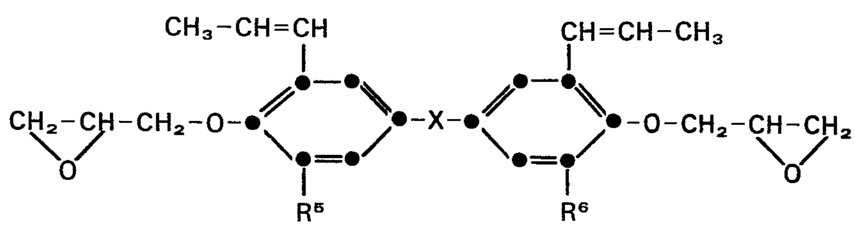

(II)

entsprechen, worin $R^5$ und $R^6$ je ein Wasserstoffatom oder eine Propenylgruppe, vorzugsweise ein Wasserstoffatom, bedeuten und X für Isopropylen, Methylen, Sulfonyl, $-O-$ oder $-S-$, vorzugsweise Isopropylen oder Methylen, steht.

Die erfindungsgemässen Verbindungen können auch in Form von Isomerengemischen, bestehend aus propenyl- und allylsubstituierten Phenolglycidyläthern, vorliegen. Darin beträgt der Anteil an Propenylgruppen vorzugsweise mindestens 10 Äquivalentprozent, insbesondere mindestens 20 Äquivalentprozent, bezogen auf die Summe der Äquivalente Propenyl- und Allylgruppen.

In einer besonders vorteilhaften Ausführungsform verwendet man solche Isomerengemische aus propenyl- und allylsubstituierten Phenolglycidyläthern, worin der Anteil an Propenylgruppen mindestens 50 Äquivalentprozent, bezogen auf die Summe aller Äquivalente Propenyl- und Allylgruppen beträgt.

Die erfindungsgemässen Phenolglycidyläther der Formel I können hergestellt werden, in dem man Phenole der Formel III:

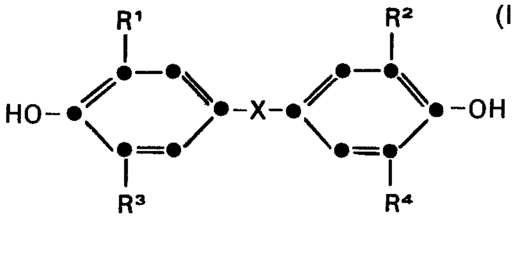

(III)

worin $R^1$, $R^2$, $R^3$ und $R^4$ die gleiche Bedeutung wie in Formel I haben, mit einem Epihalogenhydrin glycidyliert.

Verfahren zur Herstellung von Glycidyläthern von Phenolen durch katalysierte Anlagerung von Epihalogenhydrin, insbesondere Epichlorhydrin, in Gegenwart von tertiären Aminen oder quaternären Ammoniumbasen und anschliessende Dehydrohalogenierung der entstandenen Halogenhydrinäther mittels Natronlauge zu den Phenolglycidyläthern sind bekannt (siehe z.B. „Handbook of Epoxy Resins" von H. Lee und K. Neville, McGraw-Hill Book Co., New York (1967), Kapitel 2, S. 10 bis 12, und Houben-Weyl „Methoden der Organischen Chemie", Stuttgart (1963), Bd. 14, Teil 2, S. 468-470). Aus „Journal of Applied Polymer Science", Bd. IV, S. 141 bis 150, 1960, ist es ferner bekannt, dass sich alkenylsubstituierte Phenole, wie o-Allylphenol und o-Crotylphenol, auf diese Weise glycidylieren lassen.

Die bei der Glycidylierung eingesetzten propenylsubstituierten Phenole oder aus propenyl- und allylsubstituierten Phenolen bestehenden Isomerengemische können nach dem von A.R. Bader in „Journal of American Chemical Society" (1956), S. 1709, oder nach dem in „Organic Reactions" (1944), Bd. II, S. 19, beschriebenen Verfahren durch alkalische Isomerisation der entsprechenden allylsubstituierten Phenole erhalten werden, indem man zum Beispiel 2,6-Diallylphenol in Gegenwart von mindestens gleichen Mengen Kalilauge solange auf über 100° C erhitzt, bis alle Allylgruppen zu Propenylgruppen isomerisiert sind und das entsprechende 2,6-Dipropenylphenol entstanden ist. Bei Verwendung von weniger als der gleichen Menge Kalilauge, durch Anwendung von geringeren Isomerisationstemperaturen oder durch Abbrechen der Isomerisationsreaktion erreicht man, dass die Isomerisation nur partiell verläuft und man erhält somit die aus propenyl- und allylsubstituierten Phenolen bestehenden Isomerengemische. Man kann natürlich auch Isomerengemische herstellen, indem man reine Propenylphenole mit Allylphenolen mischt.

Die allylsubstituierten Phenole werden bekanntlich durch Verätherung der phenolischen Hydroxylgruppe mit Allylchlorid und anschliessende Claisen-Umlagerung erhalten. Durch Wiederholung dieser Umsetzungs- und Umlagerungsreaktion werden die entsprechenden Polyallylphenole erhalten.

Wie eingangs erwähnt, stellen die erfindungsgemässen Verbindungen und Isomerengemische wertvolle Monomere dar, die auf verschiedene Weise zu Polymeren verarbeitet werden können. Aus den erfindungsgemässen Verbindungen, insbesondere aus den Phenoldiglycidyläthern, können mit entsprechenden Vorverlängerungsmitteln, wie zweiwertigen Phenolen oder Dicarbonsäuren, nach dem sogenannten „Advancement-Verfahren" [siehe H. Batzer und S.A. Zahir in „Journal of Applied Polymer Science", *19*, S. 585, 601 und 609 (1975) und *21*, S. 1843 (1977)] vorverlängerte, propenylsubstituierte Epoxidharze hergestellt werden. So werden zum Beispiel aus n+1 mol eines propenylsubstituierten Phenoldiglycidyläthers und n mol eines zweiwertigen, gegebenenfalls alkenylsubstituierten Phenols, wobei n eine Zahl von grösser als 1 darstellt, nach dem Advancement-Verfahren höhermolekulare Epoxidharze erhalten. Unter Mitverwendung von entsprechenden Mengen o-Propenylphenol oder o-Propenylphenolglycidyläther als Kettenabbruchmittel werden nach dem Advancement-Verfahren höhermolekulare Harze erhalten, die frei von Epoxidgruppen sind, also nur Alkenylgruppen, insbesondere Propenylgruppen, als reaktive Gruppe enthalten.

Die erfindungsgemässen Verbindungen stellen insofern interessante Verbindungen dar, als sie zwei voneinander verschiedene, reaktive Gruppen aufweisen, die sowohl gleichzeitig in einer Härtungsstufe als auch nacheinander in einer Zweistufenhärtung unter Verwendung entsprechender Härtungsmittel und/oder Härtungskatalysatoren zur Vernetzung gebracht werden können.

Bei der einstufigen Härtung werden die zur Umsetzung der reaktiven Gruppen benötigten Härtungsmittel und/oder Härtungskatalysatoren gleichzeitig zugegeben. Für den Fall, dass man anionische, insbesondere aber kationische Härtungskatalysatoren verwendet, erübrigen sich weitere Härtungsmittel.

Für viele Applikationen ist die Zweistufenhärtung von besonderem Interesse, da die durch Umsetzung der einen reaktiven Gruppe erhaltenen Produkte, gegebenenfalls nach der Applikation, nachgehärtet werden können. Auf diese Weise ist es ausserdem möglich, die Endeigenschaften der gehärteten Formstoffe gewissermassen zu steuern.

So können beispielsweise aus den propenylsubstituierten Phenolglycidyläthern einerseits durch übliche Polymerisation Polymere, die Glycidylgruppen aufweisen, hergestellt werden, welche als Polyepoxide besonders für Pressmassen oder im Oberflächenschutz vorteilhaft verwendet werden können. Andererseits ist es auch möglich, in den neuen propenylsubstituierten Phenolglycidyläthern erst die Epoxidgruppe zur Reaktion zu bringen und das teilweise gehärtete, gegebenenfalls applizierte Harz durch Strahlungshärtung, zum Beispiel mit Hilfe von ionisierenden Strahlen, vollständig auszuhärten.

Als Epoxidharzhärtungsmittel kommen saure, basische und katalytische Härtungsmittel in Frage.

Als geeignete Härter seien genannt: Amine oder Amide, wie aliphatische, cycloaliphatische oder aromatische, primäre, sekundäre und tertiäre Amine, z.B. Äthylendiamin, Hexamethylendiamin, Trimethylhexamethylendiamin, Diäthylentriamin, Triäthylentetramin, N,N-Dimethylpropylendiamin-1,3, N,N-Diäthylpropylendiamin-1,3, Bis-(4-amino-3-methylcyclohexyl)methan, 3,5,5-Trimethyl-3-(aminomethyl)cyclohexylamin (Isophorondiamin), Mannichbasen, wie 2,4,6-Tris-(dimethylaminomethyl)phenol; p-Phenylendiamin, Bis-(4-aminophenyl)methan, Bis-(4-aminophenyl)sulfon, N-(2-Aminoäthyl)piperazin; Polyamide, insbesondere solche aus aliphatischen Polyaminen, wie Diäthylentriamin oder Triäthylentetramin und di- oder trimerisierten ungesättigten Fettsäuren, wie dimerisierte Leinölfettsäure (Versamid); Dicyandiamid, Anilinformaldehydharze; mehrwertige Phenole, z.B. Resorcin, 2,2-Bis-(4-hydroxyphenyl)propan oder Phenolformaldehydharze; Bortrifluorid und seine Komplexe mit organischen Verbindungen, wie $BF_3$-Äthekomplexe und $BF_3$-Aminkomplexe, z.B. $BF_3$-Monoäthylaminkomplex; Acetoacedanilid-$BF_3$-Komplex; Phosphorsäure; Triphenylphosphit; mehrbasische Carbonsäuren und ihre Anhydride, z.B. Phthalsäureanhydrid, $\Delta^4$-Tetrahydrophthalsäureanhydrid, Hexahydrophthalsäureanhydrid, 4-Methylhexahydrophthalsäureanhydrid, 3,6-Endomethylen-$\Delta^4$-tetrahydrophthalsäureanhydrid, Methyl-3,6-endomethylen-$\Delta^4$-tetrahydrophthalsäureanhydrid (=Methylnadicanhydrid), 3, 4, 5, 6, 7, 7-Hexachlor-3,6-endomethylen-$\Delta^4$-tetrahydrophthalsäureanhydrid, Bernsteinsäureanhydrid, Adipinsäureanhydrid, Azelainsäureanhydrid, Sebacinsäureanhydrid, Maleinsäureanhydrid, Dodecenylbernsteinsäureanhydrid; Pyromellithsäuredianhydrid oder Gemische solcher Anhydride.

Man kann bei der Härtung ausserdem Härtungsbeschleuniger einsetzen; bei der Verwendung von Polyamiden, Dicyandiamid oder Polycarbonsäureanhydriden als Härter eignen sich als Beschleuniger z.B. tertiäre Amine, deren Salze oder quaternäre Ammoniumverbindungen, z.B. 2,4,6-Tris-(dimethylaminomethyl)phenol, Benzyldimethylamin, 2-Äthyl-4-methylimidazol, 4-Aminopyridin, Triamylammoniumphenolat; ferner Alkalimetallalkoholate, wie z.B. Natriumhexatriolat. Bei der Aminhärtung können als Beschleuniger z.B. Mono- oder Polyphenole, wie Phenol oder Diomethan, Salicylsäure oder Rhodanide eingesetzt werden.

Zur Vernetzung der polymerisationsfähigen Doppelbindungen mittels radikalischer Polymerisation verwendet man vorzugsweise die üblichen, freie Radikale bildenden Katalysatoren; genannt seien Hydrazinderivate, z.B. Hydrazinhydrochlorid, organometallische Verbindungen, wie Tetraäthylblei, sowie insbesondere aliphatische Azoverbindungen, wie $\alpha',\alpha$-Azoisobutyrodinitril und organische Peroxide oder Persalze, wie beispielsweise Peressigsäure, Acetylperoxid, Chloroacetylperoxid , Trichloracetylperoxid, Benzoylperoxid, Chlorbenzoylperoxid, Benzoylacetylperoxid, Propionylperoxid, Fluorchlorpropionylperoxid, Laurylperoxid, Cumolhydroperoxid, Cyclohexanonhydroperoxid, tert.-Butylhydroperoxid, Di-tert.-butylperoxid, Di-tert.-amylperoxid, p-Menthanhydroperoxid; ferner anorganische Peroxidverbindungen, wie Natriumperoxid, Alkalipercarbonate, Alkalipersulfate oder Alkaliperborate, und insbesondere Wasserstoffperoxid, welches das teurere Benzoylperoxid vorteilhaft ersetzten kann.

Ausser Peroxiden eignen sich auch C-C-spaltende Initiatoren vom Dibenzyltyp zur Heisshärtung der erfindungsgemässen, ungesättigten Verbindungen. Initiatoren vom Dibenzyltyp werden von H. Wolfers *et al.* in „Kunststoffe", *68* (1978), Heft 9, S. 553 beschrieben.

Ihr Zusatz bemisst sich in bekannter Weise nach dem gewünschten Reaktionsverlauf oder den gewünschten Eigenschaften des Polymerisates. Vorteilhaft werden etwa 0,05 bis 10 Gewichtsprozente des Katalysators, berechnet auf das Gesamtgewicht der propenylsubstituierten Phenol/Monomer-Mischung, eingesetzt, wobei die Gesamtmenge des Katalysators entweder zu Beginn oder portionenweise während des Verlaufes der Polymerisation zugesetzt wird.

In gewissen Fällen können auch kationische oder anionische Katalysatoren verwendet werden, mit denen auch die Epoxidgruppe zur Reaktion gebracht werden kann.

Die erfindungsgemässen propenylsubstituierten Phenolglycidyläther können sowohl allein oder in Mischung mit anderen Epoxidharzen und vorzugsweise mit anderen polymerisierbaren Monomeren unter Formgebung oder als Flächengebilde ausgehärtet und/oder polymerisiert bzw. copolymerisiert werden. Die erfindungsgemässen Verbindungen zeigen eine sehr gute Verträglichkeit und Mischbarkeit mit anderen polymerisierbaren Monomeren, wobei mengenmässig relativ viel dieser Monomeren verwendet werden können, ohne dass die mechanischen Eigenschaften der daraus hergestellten Formkörper sich verschlechtern.

Die erfindungsgemässen propenylsubstituierten Phenolglycidyläther bzw. deren härtbaren Mischungen können ferner vor der Härtung in irgendeiner Phase mit üblichen Modifizierungsmitteln, wie Streck-, Füll- und Verstärkungsmitteln, Pigmenten, Farbstoffen, organischen Lösungsmitteln, Weichmachern, Verlaufmitteln, Thixotropiermitteln, flammhemmenden Stoffen oder Formtrennmitteln, versetzt werden.

Die härtbaren, propenylsubstituierten Phenolglycidyläther enthaltenden Mischungen finden ihren Einsatz vor allem auf den Gebieten des Oberflächenschutzes, der Elektrotechnik, der Laminierverfahren und im Bauwesen. Sie können in jeweils dem speziellen Anwendungszweck angepasster Formulierung, im ungefüllten oder gefüllten Zustand, gegebenenfalls in Form von Lösungen oder Emulsionen, als Anstrichmittel, Lacke, als Pressmassen, Sinterpulver, Tauchharze, Giessharze, Spritzgussformulierungen, Imprägnierharze und Bindemittel, Klebstoffe, als Werkzeugharze, Laminierharze, Dichtungs- und Spachtelmassen, Bodenbelagsmassen und Bindemittel für mineralische Aggregate, verwendet werden.

In den nachfolgenden Beispielen bedeuten, wenn nichts anderes angegeben ist, Teile Gewichtsteile.

*Beispiel 1:*

a) *Herstellung von 2,2-Bis-[3-(1-propenyl)-4-hydroxyphenyl]propan*

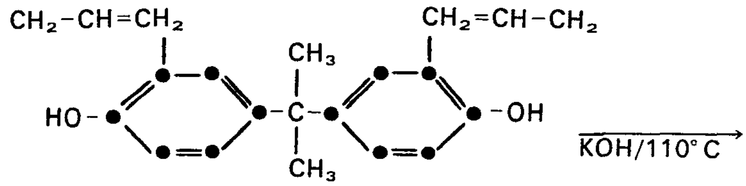


($A_1$)

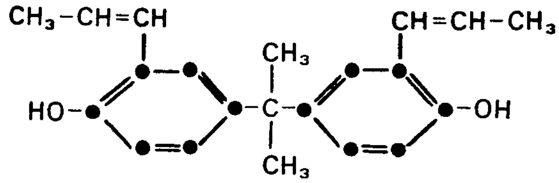

($A_2$)

Unter Rühren werden 400 ml Methanol langsam zu einer Mischung aus 3 mol 2,2-Bis-(3-allyl-4-hydroxyphenyl)propan ($A_1$) und Kaliumhydroxidkügelchen zugegeben. Danach wird die Mischung sorgfältig auf 110° C unter Abdestillieren von 116 ml Methanol erhitzt. Anschliessend wird die Reaktionslösung unter Rückflusskühlung noch weitere 6 h auf 110° C gehalten. Dann wird die Reaktionslösung gekühlt, mit konz. HCl neutralisiert und mit Methylenchlorid extrahiert. Der Extrakt wird getrocknet und vollständig zur Trokkene eingedampft. Man erhält im wesentlichen reines 2,2-Bis-[3-(1-propenyl)-4-hydroxyphenyl)propan ($A_2$), das bei Raumtemperatur eine sehr hochviskose, gelbliche Flüssigkeit darstellt. Durch Mikroanalyse, H–NMR-, MS- und UV-Spektroskopie sowie mittels Gelpermeationschromatographie wird die Strukturformel $A_2$ bestätigt.

b) *Herstellung von 2,2-Bis-[3-(1-propenyl)-4-glycidyloxyphenyl]propan*

20 mol Epichlorhydrin werden zu 2 mol 2,2-Bis-[3-(1-propenyl)-4-hydroxyphenyl]propan zugegeben und die Lösung wird auf 90° C erhitzt. Dann werden 5 g einer 1N-Natronlauge und 10 g einer 50%igen wässrigen Tetramethylammoniumchloridlösung zugegeben und die Lösung wird während 90 Min unter Rühren auf 90° C erhitzt, wobei eine schwache exotherme Reaktion auftritt. Dann wird die Lösung auf 60° C gekühlt und im Vakuum von 93 bis 119 mbar unter Rückfluss, wobei der Rückflusskühler einen Wasserabscheider (Dean-Stark-Falle) aufweist, gebracht. Dann werden langsam 352 g einer 50%igen wässrigen Natronlauge zugegeben und das Wasser kontinuierlich bei 60° C durch azeotrope Kreislaufdestillation aus der Reaktionslösung entfernt. Nach 2 h ist das Wasser abgeschieden und die Lösung wird für weitere 2 h bei 90° C gerührt. Dann wird die Lösung auf 30° C gekühlt und vom Salz abfiltriert. Das Filtrat wird in Methylenchlorid aufgenommen und wiederholt mit einer 5%igen wässrigen $Na_2HPO_4$-Lösung und Wasser gewaschen. Die organische Phase wird abgetrennt und über $Na_2SO_4$ getrocknet. Im Rotationsverdampfer wird die Reaktionslösung von flüchtigen Anteilen befreit. Es wird ein viskoses Produkt mit einem Epoxidgehalt von 4,32 Äquivalenten/kg (Theorie: 4,76 Äquivalente/kg) und einer Viskosität von $\eta$ 40° C = 59870 mPa/s erhalten.

H–NMR-, MS- und UV-Spektren bestätigen, dass dem erhaltenen Produkt die folgende Strukturformel zukommt.

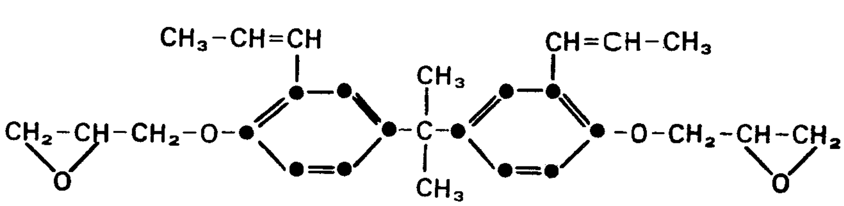

*Anwendungsbeispiele*

*Beispiele I und II:* 2,2-Bis-[3-(1-propenyl)-4-glycidyloxyphenyl]propan mit einem Epoxidgehalt von 4,46 Äquivalenten/kg [o,o'-Di-(1-propenyl)bisphenol A] und 2,2-Bis-(3-allyl-4-glycidyloxyphenyl)propan mit einem Epoxidgehalt von 4,53 Äquivalenten/kg (o,o'-Diallylbisphenol A) werden mit Hexahydrophthalsäureanhydrid bzw. 4,4'-Diaminodiphenylmethan in den in Tabelle 1 angegebenen Mengen vermischt und ausgehärtet. Die Eigenschaften der erhaltenen Formstoffe sind in Tabelle 1 angegeben.

*Tabelle 1*

| | Beispiel I | Vergleich 1 | Beispiel II | Vergleich ' |
|---|---|---|---|---|
| o,o'-Di-(1-propenyl)bisphenol A (Teile) | 100 | | 100 | |
| o,o'-Diallylbisphenol A (Teile) | | 100 | | 100 |
| Hexahydrophthalsäureanhydrid (Teile) | 59,25 | 62,5 | | |
| 4,4'-Diaminodiphenylmethan (Teile) | | | 21,9 | 23,1 |
| Benzyldimethylamin (Teile) | 0,5 | 0,5 | | |
| Härtungsbedingungen | 4 h bei 80° C und 8 h bei 140° C | | | |
| Biegefestigkeit nach Dynstat in $N/m^2 \times 10^8$ | 1,432 | 1,322 | 1,704 | 1,257 |
| Glasumwandlungstemperatur $T_G$ (° C) | 118 | 97,5 | 147 | 120 |

*Beispiele III bis VI:* 2,2-Bis-[3-(1-propenyl)-4-glycidyloxyphenyl]propan mit einem Epoxidgehalt von 4,46 Äquivalenten/kg [o,o'-Di-(1-propenyl)bisphenol A] und 2,2-Bis-(3-allyl-4-glycidyloxyphenyl)propan mit einem Epoxidgehalt von 4,53 Äquivalenten/kg (o,o'-Diallylbisphenol A) werden mit 2-Äthyl-4-methylimidazol bzw. Maleinsäureanhydrid in den in Tabelle 2 angegebenen Mengen gut vermischt. Aus diesen Mischung werden gemäss Vorschrift des Twist-O-Meters [der Firma Epprecht Instruments und Controls, Bassersdorf (CH)] Aluminiumzapfen verklebt, indem man die Klebschicht bei den in Tabelle 2 angegebenen Temperaturen aushärtet. Die Ergebnisse der Torsionsscherfestigkeitsmessungen sind in Tabelle 2 angegeben.

*Tabelle 2*

| | Beispiel III | Beispiel IV | Vergleich 3 | Vergleich 4 | Beispiel V | Beispiel VI | Vergleich 5 | Vergleich 6 |
|---|---|---|---|---|---|---|---|---|
| o,o'-Di-(1-propenyl-bisphenol A (Teile) | 100 | 100 | | | 100 | 100 | | |
| o,o'-Diallylbisphenol A (Teile) | | | 100 | 100 | | | 100 | 100 |
| 2-Äthyl-4-methylimidazol (Teile) | 3 | 3 | 3 | 3 | | | | |
| tert.-Butylperbenzoat | | 3 | | 3 | | | | |
| Maleinsäureanhydrid (Teile) | | | | | 36,0 | 36,0 | 37,7 | 37,3 |
| Benzyldimethylamin (Teile) | | | | | | 4 | | 4 |
| Härtungsbedingungen | 1 h bei 80° C, 3 h bei 150° C und 2 h bei 200° C | | | | 4 h bei 80° C und 8 h bei 150° C | | | |
| Torsionsscherfestigkeit ($N/m^2 \times 10^6$) | 97,2 | 96,8 | 73,0 | 70,8 | 32,6 | 88,4 | 4,0 | 71,4 |
| Glasumwandlungs-temperatur $T_G$ (° C) | 136 | | 102 | | | | | |

## Patentansprüche

1. Phenolglycidyläther der Formel I:

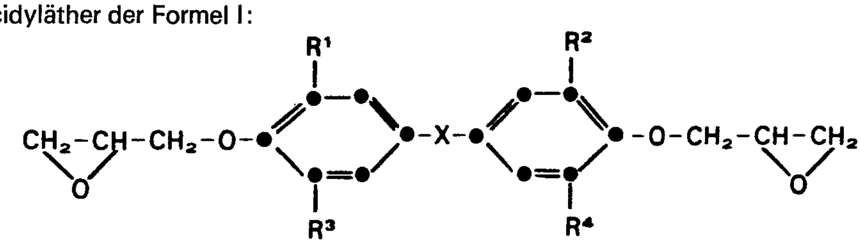

(I)

worin $R^1$, $R^2$, $R^3$ und $R^4$ je ein Wasserstoffatom, eine Allyl- oder Propenylgruppe bedeuten, wobei mindestens einer der Substituenten $R^1$ bis $R^4$ für die Propenylgruppe steht, und X Isopropylen, Methylen, Sulfonyl, –O– oder –S– bedeutet.

2. Phenolglycidyläther gemäss Anspruch 1 der Formel II:

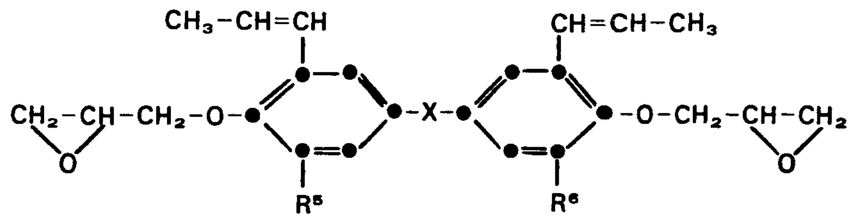

(II)

worin $R^5$ und $R^6$ je ein Wasserstoffatom oder eine Propenylgruppe bedeuten und X die gleiche Bedeutung wie in Formel I hat.

3. 2,2-Bis-[3-(1-propenyl)-4-glycidyloxy-phenyl]propan als Verbindung der Formel II gemäss Anspruch 2.

4. Verfahren zur Herstellung von Phenolglycidyläthern der Formel I gemäss Anspruch 1, dadurch gekennzeichnet, dass man Phenole der Formel III:

(III)

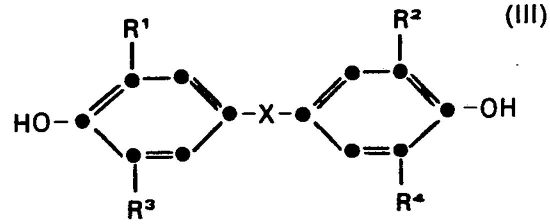

worin $R^1$, $R^2$, $R^3$ und $R^4$ die gleiche Bedeutung wie in Formel I haben, mit einem Epihalogenhydrin glycidyliert.

5. Verwendung der Phenolglycidyläther der Formel I gemäss Anspruch 1 zur Herstellung von Polymeren.

---

## Claims

1. A phenolglycidyl ether of the formula I

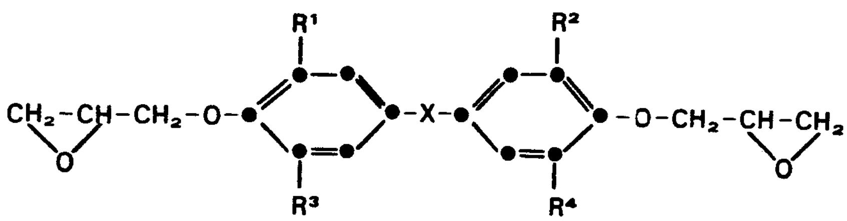

(I)

wherein $R^1$, $R^2$, $R^3$ and $R^4$ are each a hydrogen atom, or an allyl or propenyl group, with at least one of the substituents $R^1$ to $R^4$ being the propenyl group, and X is isopropylene, methylene, sulfonyl, –O– or –S–.

2. A phenolglycidylether according to Claim 1 of the formula II

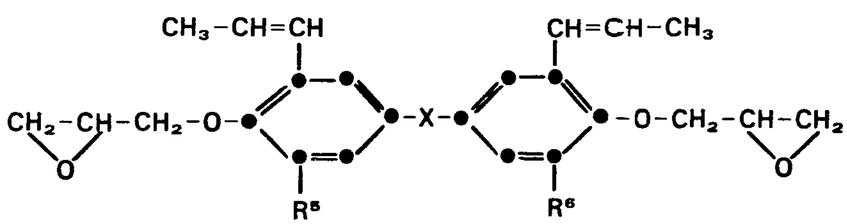

(II)

wherein $R^5$ and $R^6$ are each a hydrogen atom or a propenyl group, and X has the same meaning as in the formula I.

3. 2,2-Bis-[3-(1-propenyl)-4-glycidyloxy-phenyl]-propane as a compound of the formula II according to Claim 2.

4. A process for producing a phenolglycidylether of the formula I according to Claim 1, which process comprises glycidylating a phenol of the formula III

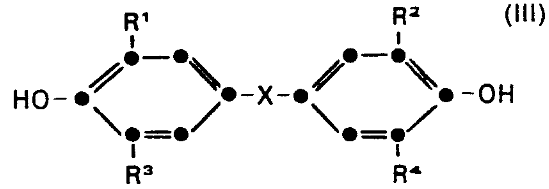

(III)

wherein $R^1$, $R^2$, $R^3$ and $R^4$ have the same meaning as in the formula I, with an epihalohydrine.

5. Use of a phenolglycidylether of the formula I according to Claim 1 for producing polymers.

---

**Revendications**

1. Ethers glycidyliques de phénols répondant à la formule I:

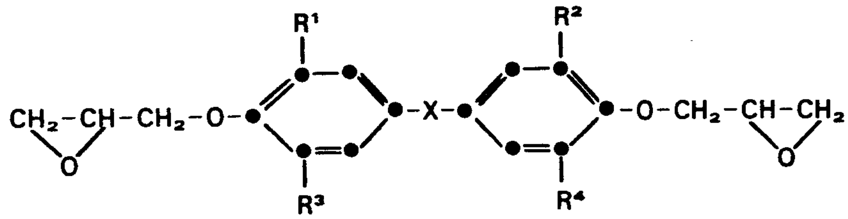

(I)

dans laquelle $R^1$, $R^2$, $R^3$ et $R^4$ représentent chacun un atome d'hydrogène, un radical allyle ou un radical propényle, au moins l'un des substituants $R^1$ à $R^4$ étant un radical propényle, et X représente un radical isopropylène, méthylène, sulfonyle, −O− ou −S−.

2. Ethers glycidyliques de phénols selon la revendication 1 qui répondent à la formule II:

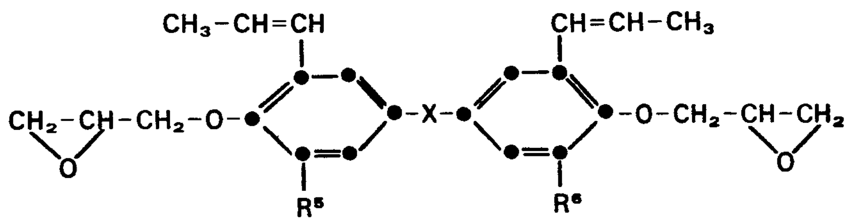

(II)

dans laquelle $R^5$ et $R^6$ représentent chacun un atome d'hydrogène ou un radical propényle et X a la même signification que dans la formule I.

3. En tant que composé de formule II selon la revendication 2, le bis-[(propène-1 yl)-3 glycidyloxy-4 phényl]-2,2 propane.

4. Procédé de préparation d'éthers glycidyliques de phénols de formule I selon la revendication 1, caractérisé en ce qu'on glycidyle, au moyen d'une épihalogénhydrine, des phénols répondant à la formule III:

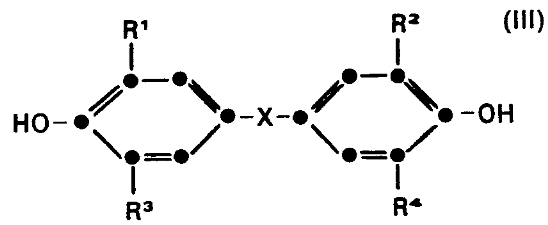

(III)

dans laquelle $R^1$, $R^2$, $R^3$ et $R^4$ ont les mêmes significations que dans la formule I.

5. Application des éthers glycidyliques de phénols de formule I selon la revendication 1 à la préparation de polymères.